# EUROPEAN PATENT SPECIFICATION

(11) **EP 3 240 628 B1**
(45) Date of publication and mention of the grant of the patent: **14.07.2021**
(21) Application number: 15817880.6
(22) Date of filing: 28.12.2015
(51) Int. Cl.: B01J 13/00

(54) **CARBOXYL CROSS-LINKED CHONDROITIN HYDROGELS AND THEIR USE FOR SOFT TISSUE APPLICATIONS**
CARBOXYLVERNETZTE CHONDROITINHYDROGELE UND DEREN VERWENDUNG FÜR WEICHGEWEBEANWENDUNGEN
HYDROGELS DE CHONDROÏTINE RÉTICULÉE CARBOXYLE ET LEUR UTILISATION POUR DES APPLICATIONS DE TISSU MOU

(30) Priority: 29.12.2014 EP 14200385
(43) Date of publication of application: 08.11.2017
(73) Proprietor: Galderma S.A., 6330 Cham (CH)
(72) Inventor: Karlsson Anders, 743 40 Storvreta (SE); Mojarradi, Hotan, 754 31 Uppsala (SE)
(74) Representative: AWA Sweden AB
(86) International application number: PCT/EP2015/081266
(87) International publication number: WO 2016/107834

(56) References cited:
- WO-A1-2013/109959
- STREHIN I ET AL: "A versatile pH sensitive chondroitin sulfate@?PEG tissue adhesive and hydrogel", BIOMATERIALS, ELSEVIER SCIENCE PUBLISHERS BV., BARKING, GB, vol. 31, no. 10, 1 April 2010 (2010-04-01) , pages 2788-2797, XP026882486, ISSN: 0142-9612, DOI: 10.1016/J.BIOMATERIALS.2009.12.033 [retrieved on 2010-01-04]
- None

## Description

### Technical field of the invention

The present invention relates to the field of hydrogels containing cross-linked polysaccharides and the use of such hydrogels in medical and/or cosmetic applications. More specifically, the present invention is concerned with hydrogels made of cross-linked chondroitin or chondroitin sulfate.

### Background to the invention

Water-absorbing gels, or hydrogels, are widely used in the biomedical field. They are generally prepared by chemical crosslinking of polymers to infinite networks. While many polysaccharides absorb water until they are completely dissolved, cross-linked gels of the same polysaccharides can typically absorb a certain amount of water until they are saturated, i.e. they have a finite liquid retention capacity, or swelling degree.

Chondroitin and chondroitin sulfate are well-known biocompatible polymer. They are naturally occurring polysaccharides belonging to the group of glycosaminoglycans (GAGs) together with e.g. hyaluronic acid. All GAGs are negatively charged heteropolysaccharide chains which have a capacity to absorb large amounts of water. Hyaluronic acid is widely used in the biomedical and cosmetic fields, for instance during viscosurgery and as a dermal filler.

Chondroitin sulfate (CS) is a highly abundant GAG found in the connective tissues of mammals where it, together with other sulfated GAGs, is bound to proteins as part proteoglycans. It has previously been shown that hydrogels containing CS successfully can be used in biomedical applications due to their resemblance to the natural extra cellular matrix (Lauder, R.M., Complement Ther Med 17: 56-62, 2009). Chondroitin sulfate is also used in the treatment of osteoarthritis, e.g. as a dietary supplement.

Strehin et al., Biomaterials 31: 2788-2797 (2010) discloses grafting of polyethylene glycol (PEG) amine to chondroitin sulfate (CS) using EDC/NHS chemistry. The reaction is inefficient and prone to produce undesirable impurities due to side reactions.

WO 2013/109959 A1 discloses nanoparticles made of chondroitin sulfate subjected to EDC/dihydrazide chemistry. However, no stable 3-D structure network was formed with chondroitin sulfate. Again, the reaction is inefficient and prone to produce undesirable impurities due to side reactions.

### Summary of the invention

It is an object of the present invention to provide a hydrogel having a chondroitin molecule as the swellable polymer.

It is also an object of the present invention to provide a method for preparing hydrogels of chondroitin molecules by mild and efficient routes.

It is one object of the present invention to provide a method for preparing hydrogels of chondroitin molecules with a high yield. It is a further object of the present invention to provide a method for preparing hydrogels of chondroitin molecules with a low degree of impurities.

For these and other objects that will be evident from this disclosure, the present invention provides according to a first aspect a process of preparing a hydrogel product comprising a cross-linked network of chondroitin molecules, comprising the steps of:
(a) providing chondroitin molecules selected from the group consisting of chondroitin and chondroitin sulfate;
(b) activating the carboxyl groups on the chondroitin molecules with a triazine-based coupling reagent to form an activated, cross-linked chondroitin essentially without any N-acylurea groups;
(c) cross-linking the activated chondroitin molecules via their carboxyl groups using a cross-linking agent, wherein the crosslinking agent is hexamethylenediamine (HMDA).

The present invention involves cross-linking of chondroitin molecules by covalent bonds, preferably amide bonds, typically using a triazine-based activating agent for the carboxyl groups on the chondroitin molecule backbone and a multi-functional cross-linking agent. Cross-linking is achieved by mild and efficient routes, in high yield and with a low degree of impurities. The mild reaction which is enabled by the triazine-based coupling reagents is particularly important for chondroitin sulfate, which is more sensitive due to risk of de-sulfatation.

In a certain embodiments, the activation step (b) and the cross-linking step (c) occur simultaneously. In other embodiments, the activation step (b) occurs prior to and separately from the cross-linking step (c).

In a preferred embodiment, step (c) further comprises providing particles of the cross-linked chondroitin molecule, having an average size in the range of 0.01-5 mm, preferably 0.1-0.8 mm.

Preferred triazine-based coupling reagents are 2-chloro-4,6-disubstituted-1,3,5-triazines in the presence of a tertiary amine base or the corresponding quarternary ammonium salts thereof, such as 2-chloro-4,6-dimethoxy-1,3,5-triazin (CDMT) together with N-methylmorpholine (NMM), or 4-(4,6-dimethoxy-1,3,5-triazin-2-yl)-4-methylmorpholinium chloride (DMTMM), preferably DMTMM.

The cross-linking agent of step (c) is hexamethylenediamine (HMDA).

The present invention provides according to a second aspect a hydrogel product comprising a cross-linked network of chondroitin molecules selected from the group consisting of chondroitin and chondroitin sulfate as the swellable polymer, wherein the carboxyl groups on the chondroitin molecules are activated using a triazine-based coupling agent and wherein the chondroitin molecules are cross-linked to each other via their carboxyl groups, essentially without any N-acylurea groups on the chondroitin molecules; wherein the crosslinking agent is hexamethylenediamine (HMDA). This cross-linked product thus has a low degree of impurities.

The present invention involves chondroitin molecules cross-linked by covalent amide bonds.

In an embodiment, the cross-linked chondroitin molecule is in the form of gel particles having an average size in the range of 0.01-5 mm, preferably 0.1-0.8 mm. The hydrogel product may have the form of an injectable formulation.

In one embodiment, the hydrogel product is obtainable, or obtained, by the process according to the invention.

According to related aspect, the present invention also provides use of the hydrogel product as a medicament, such as in the treatment of soft tissue disorders. There is provided a method of treating a patient suffering from a soft tissue disorder by administering to the patient a therapeutically effective amount of the hydrogel product. There is also provided a method of providing corrective or aesthetic treatment to a patient by administering to the patient a therapeutically effective amount of the hydrogel product.

Other aspects and preferred embodiments of the present invention will be evident from the following detailed disclosure of the invention and the appended claims.

### Brief description of the drawings

Fig. 1 shows the structure of the chondroitin (CN) disaccharide repeating unit consisting of alternating non-sulfated D-glucuronic acid and N-acetyl-D-galactosamine moieties.
Fig. 2 shows the structure of the chondroitin sulfate (CS) disaccharide repeating unit consisting of alternating D-glucuronic acid and N-acetyl-D-galactosamine moieties, also showing the sulfation pattern where the hydroxyl group(s) is (are) substituted by a sulfate group(s) (R=SO₃H).
Fig. 3 is a general scheme illustrating the cross-linking route, involving covalent binding of bifunctional nitrogen-based crosslinking agents to native carboxylic groups on the glucuronic acid moieties of chondroitin sulfate by use of a coupling agent.

### Detailed description of the invention

The present invention provides advantageous processes for preparing hydrogels made of cross-linked networks of chondroitin molecules, the resulting hydrogel products and uses thereof. In the hydrogel products according to the invention, the cross-linked chondroitin molecule is the swellable polymer which provides the gel properties. For avoidance of doubt, the cross-linked hydrogel does not contain any further polymer. The preparation process described herein is mild to the chondroitin molecules but provides an efficient cross-linking, e.g. a high yield.

Thus, the current invention provides chondroitin molecule hydrogels by crosslinking chondroitin molecules to each other in aqueous media using dual-nucleophilic functional molecules capable of forming covalent bonds directly with carboxylic acid groups of the glucuronic acid moiety on chondroitin molecules by a reaction involving the use of a coupling agent.

The chondroitin molecules according to the invention are selected from the group consisting of chondroitin and chondroitin sulfate. In a preferrred embodiment, the chondroitin molecule is chondroitin. In another preferrred embodiment, the chondroitin molecule is chondroitin sulfate. The mild reaction which is enabled by the triazine-based coupling reagents is particularly important for chondroitin sulfate, which is more sensitive due to risk of de-sulfatation.

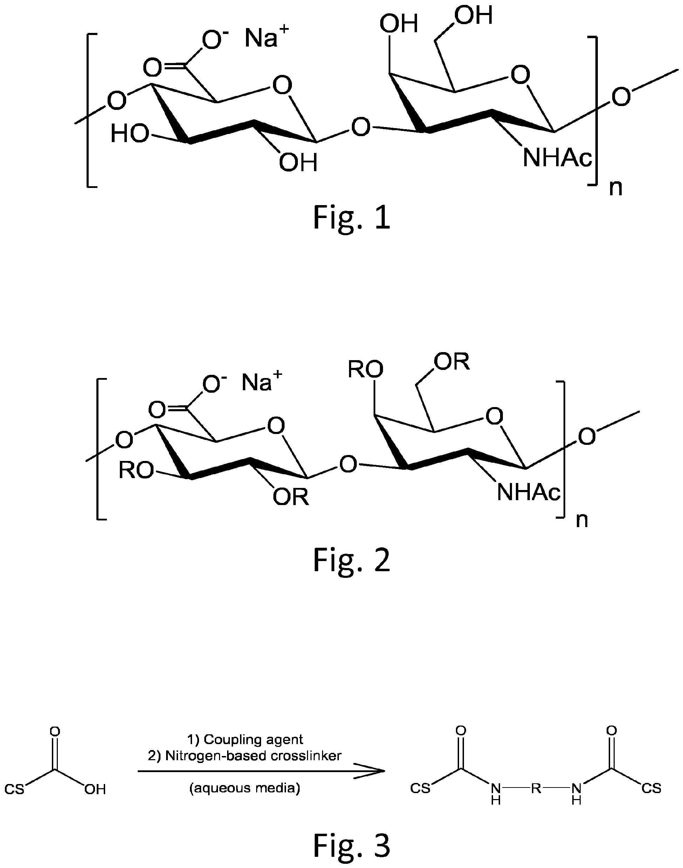

The term "chondroitin" refers to GAGs having as a disaccharide repeating unit (CN) the general structure shown in Fig. 1, consisting of alternating non-sulfated D-glucuronic acid and N-acetyl-D-galactosamine moieties. For avoidance of doubt, the term "chondroitin" does not encompass any form of chondroitin sulfate.

The term "chondroitin sulfate" refers to GAGs having as a disaccharide repeating unit (CS) the general structure shown in Fig. 2, consisting of alternating D-glucuronic acid and N-acetyl-D-galactosamine moieties. As shown in Fig. 2, the at least one sulfate moiety can be present in various different positions. Preferred chondroitin sulfate molecules are chondroitin-4-sulfate and chondroitin-6-sulfate.

The chondroitin molecules can be obtained from various sources of animal and non-animal origin. Sources of non-animal origin include yeast and preferably bacteria. The molecular weight of a single chondroitin molecule is typically in the range of 1-500 kDa, but other molecular weights are possible.

A crosslinked chondroitin molecule comprises cross-links between the chondroitin molecule chains, which creates a continuous network of chondroitin molecules which is held together by the covalent cross-links, physical entangling of the chondroitin molecule chains and various interactions, such as electrostatic interactions, hydrogen bonding and van der Waals forces.

The chondroitin molecule is cross-linked by amide bonds.

The cross-linked chondroitin molecule product is preferably biocompatible. This implies that no, or only very mild, immune response occurs in the treated individual. That is, no or only very mild undesirable local or systemic effects occur in the treated individual. It is therefore advantageous that the cross-linked product contains a a low degree of impurities. Specifically, when reacting EDC with a carboxylic acid, an intermediate O-acylurea is formed which further reacts with amines forming amide bonds. However, due to rearrangement of the O-acylurea a more stable N-acylurea is also formed which will not enable amide bond formation in the presence of amines. Using triazine-based coupling reagents such as DMTMM, the chondroitin molecules are cross-linked to each other via their carboxyl groups without any formation of N-acylurea groups or other undesirable impurities. This cross-linked product thus has a low degree of impurities.

The cross-linked chondroitin molecule product according to the invention may be a gel, or a hydrogel. That is, it can be regarded as a water-insoluble, but substantially dilute crosslinked system of chondroitin molecules when subjected to a liquid, typically an aqueous liquid.

The gel contains mostly liquid by weight and can e.g. contain 90-99.9%, water, but it behaves like a solid due to a three-dimensional cross-linked chondroitin molecule network within the liquid. Due to its significant liquid content, the gel is structurally flexible and similar to natural tissue, which makes it very useful as a scaffold in tissue engineering and for tissue augmentation. It is also useful for treatment of soft tissue disorder and for corrective or aesthetic treatment. It is preferably used as an injectable formulation.

Cross-linking of the chondroitin molecule is achieved by activation with a coupling agent, followed by reaction with a cross-linking agent. The chondroitin molecule concentration and the extent of cross-linking affect the mechanical properties, e.g. the elastic modulus G', and stability properties, of the gel. Cross-linked chondroitin molecule gels can be characterized in terms of "degree of modification". The degree of modification of chondroitin molecule gels generally range between 0.1 and 15 mole%. The degree of modification (mole%) describes the amount of cross-linking agent(s) that is bound to the chondroitin molecule, i.e. molar amount of bound cross-linking agent(s) relative to the total molar amount of repeating CS/CN disaccharide units. The degree of modification reflects to what degree the chondroitin molecule has been chemically modified by the cross-linking agent. Reaction conditions for activation and cross-linking and suitable analytical techniques for determining the degree of modification are all well known to the person skilled in the art, who easily can adjust these and other relevant factors and thereby provide suitable conditions to obtain a desirable degree of modification and verify the resulting product characteristics with respect to the degree of modification.

Chondroitin molecule gels may also comprise a portion of chondroitin molecules which are not cross-linked, i.e not bound to the three-dimensional cross-linked chondroitin molecule network. However, it is preferred that at least 50 % by weight, preferably at least 60 % by weight, more preferably at least 70 % by weight, and most preferably at least 80 % by weight, of the chondroitin molecules in a gel composition form part of the cross-linked chondroitin molecule network.

The cross-linked chondroitin molecule is preferably present in the form of gel particles. The gel particles preferably have an average size in the range of 0.01-5 mm, preferably 0.1-0.8 mm, such as 0.2-0.5 mm or 0.5-0.8 mm.

The hydrogel product may be present in an aqueous solution, but it may also be present in dried or precipitated form, e.g. in ethanol. The hydrogel product is preferably injectable.

Since the nature of the product obtainable by the processes according to the invention is complex, the product may also be defined as being the result of these processes i.e. obtained or obtainable thereby.

The hydrogel product may prepared by a process comprising the steps of:
(a) providing chondroitin molecules selected from the group consisting of chondroitin and chondroitin sulfate;
(b) activating the carboxyl groups on the chondroitin molecules with a triazine-based coupling reagent to form an activated, cross-linked chondroitin essentially without any N-acylurea groups;
(c) cross-linking the activated chondroitin molecules to each other via their carboxyl groups using a cross-linking agent, wherein the crosslinking agent is hexamethylenediamine (HMDA) thus forming a cross-linked network of chondroitin molecules.

The chondroitin molecules according to the invention are selected from the group consisting of chondroitin and chondroitin sulfate. In a preferrred embodiment, the chondroitin molecule is chondroitin. In another preferrred embodiment, the chondroitin molecule is chondroitin sulfate.

In the activation step (b), the carboxyl groups on the chondroitin molecules are activated with a triazine-based coupling reagent to form an activated, cross-linked chondroitin.

The triazine-based coupling reagent is preferably selected from 2-chloro-4,6-disubstituted-1,3,5-triazines in the presence of a tertiary amine base or the corresponding quarternary ammonium salts thereof, such as 4-(4,6-dimethoxy-1,3,5-triazin-2-yl)-4-methylmorpholinium chloride (DMTMM) and 2-chloro-4,6-dimethoxy-1,3,5-triazine (CDMT) together with N-methylmorpholine (NMM. A preferred triazine-based coupling reagent is DMTMM.

In the cross-linking step (c), cross-linking of the activated chondroitin molecules occurs via their carboxyl groups using a cross-linking agent. The cross-linking agent of step (c) is hexamethylenediamine (HMDA).

The process can advantageously be performed in a one-pot approach in aqueous media, involving the covalent coupling of bifunctional, nitrogen-based crosslinkers directly to inherent carboxylic acid groups on the native polysaccharides using a suitable triazine-based coupling reagent. In a preferred embodiment, the activation step (b) and the cross-linking step (c) occur simultaneously.

In another embodiment, the activation step (b) occurs prior to and separately from the cross-linking step (c).

The process for generating the crosslinked hydrogel typically involves preparing a mixture of a chondroitin molecule (having a molecular weight of 1- 500 kDa with preference to 10 - 200 kDa or even more preferred 20 - 100 kDa, at a concentration of 10 - 500 mg/mL with preference to 100 - 500 mg/mL) together with a cross-linking agent, being hexamethylenediamine (0.01 - 10 molar equivalents of amine towards carboxylic acid groups, or preferably 0.1 - 1 molar equivalents) and a triazine-based coupling reagent such as DMTMM (0.01 - 10 molar equivalents to carboxylic acid groups, or preferably 0.1 - 1 molar equivalents). Incubating the mixture at 5 - 50 °C, with preference to 10 - 40 °C or even more preferred 20 - 30 °C, during 2 - 120 hours, with preference to 4 - 80 hours, yields a hydrogel. The hydrogel is preferably micronized to hydrogel particles in the size of 0.01 - 5 mm, preferably 0.1-1 mm, that subsequently are washed in excess aqueous solution such as phosphate buffered saline, sodium chloride or deionized water, followed by precipitation in ethanol and drying in vacuum.

A typical application of the resulting macromolecular structures involves the preparation of injectable formulations for treatment of soft tissue disorders, including, but not limited to, corrective and aesthetic treatments.

Without desiring to be limited thereto, the present invention will in the following be illustrated by way of examples.

### Examples

### Example 1 - Triazine-activated crosslinkinq with dihydrazides (not according to the invention)

A mixture of chondroitin sulfate (CS), succinic dihydrazide (SDH), 4-methylmorpholine (NMM) and 2-chloro-4,6-1,3,5-triazine (CDMT) in a 3:2 mixture of water/acetonitrile was vigorously shaken for 2 minutes, followed by incubation at room temperature for 17 hours (see Table 1 for details).

A solid gel had then formed that was reduced to gel particles (by pressing through a filter pore-size 315 µm), which were washed and precipitated in water and ethanol and dried for 16 hours at 150 mbar and 25 °C.

¹H-NMR analysis of enzymatically degraded gel particles showed a modification degree of 2.6%.

**Table 1 - Reaction parameters for trizaine-activated crosslinking of chondroitin sulfate with dihydrazide**

| Concentration CS | Molar ratio CDMT/CS (disaccharide) | Molar ratio SDH/CDMT | Molar ratio NMM/CDMT |
|---|---|---|---|
| 20 % | 0.1 | 1.00 | 1.20 |

### Example 2 - Triazine-activated cross-linking with diamines

A series of experiments were performed which involved cross-linking of chondroitin sulfate (CS) with hexamethylenediamine (HMDA) using 4-(4,6-dimethoxy-1,3,5-triazin-2-yl)-4-methylmorpholinium chloride (DMTMM). A mixture of CS, HMDA and DMTMM in water was vortexed until a clear solution was obtained (see Table 2 for details).

The solution was incubated at 23-50 °C during 6-72 hours and the resulting gels reduced to gel particles (by pressing through a filter pore-size 315 µm), which were diluted in 0.9 % sodium chloride and then washed and precipitated in ethanol and finally dried in vacuum over night.

¹H-NMR analysis of enzymatically degraded gels (chondroitinase) showed that the degree of modification varied depending on the reaction parameters, from 24 - 43 % for gels prepared using 0.4 - 0.7 equivalents of triazine to CS disaccharides respectively, as determined by comparing integrated signals from HMDA (-CH₂- closest to the amide bond) and the N-acetyl group of CS (-CH₃).

Rheological experiments were further carried out to confirm the formation of gels by comparison of storage and loss modulus (G'>G").

**Table 2. Reaction parameters for trizaine-activated crosslinking of chondroitin sulfate with diamine**

| Concentration CS | Molar ratio DMTMM/CS (disaccharide) | Molar ratio HMDA/CS (disaccharide) |
|---|---|---|
| 16.7 - 50 % | 0.4 - 0.7 | 1 -2 |

The yield of cross-linked chondroitin sulfate in the formed gel using the triazine activation was high, 60%.

### Comparative Example 3 - Carbodiimide-activated cross-linking with diamines

As a comparison, the crosslinking efficiency for chondroitin sulfate was evaluated with a diamine using another activation agent, carbodiimide.

### Activation

14 volumes of 10% chondroitin sulfate, 3 volumes of 67% EDC (N-(3-Dimethylaminopropyl)-N'-ethylcarbonate and 3 volumes of 25 % NHS (N-hydroxysuccinimide were mixed, all solutions dissolved in PBS buffer pH 7, the activation was done at 37 °C for 10 minutes. The solution was frozen, precipitated with cold ethanol and purified by washing with cold ethanol. The activated chondroitin sulfate was then dried in a vacuum chamber.

### Cross-linking

1 volume of 10% activated chondroitin sulfate was mixed with 1 volume of 0.23% HMDA (hexamethylene diamine hydrochloride), both dissolved in PBS pH 7. The cross-linking reaction was performed at 37 °C for 10 minutes and 360 minutes, respectively. The formed gels were washed with PBS twice, precipitated with ethanol and dried. The yield of cross-linked chondroitin sulfate in the formed gel was 25 and 30%, respectively.

## Claims

1. A process of preparing a hydrogel product comprising a cross-linked network of chondroitin molecules, comprising the steps of:
(a) providing chondroitin molecules selected from the group consisting of chondroitin and chondroitin sulfate;
(b) activating the carboxyl groups on the chondroitin molecules with a triazine-based coupling reagent to form an activated, cross-linked chondroitin essentially without any N-acylurea groups;
(c) cross-linking the activated chondroitin molecules to each other via their carboxyl groups using a cross-linking agent, wherein the crosslinking agent is hexamethylenediamine (HMDA).

2. A process according to claim 1, wherein the cross-linking of step (c) provides amide bonds.

3. A process according to any one of claims 1-2, wherein the triazine-based coupling reagent is selected from 2-chloro-4,6-disubstituted-1,3,5-triazines in the presence of a tertiary amine base or the corresponding quarternary ammonium salts thereof.

4. A process according to claim 3, wherein the triazine-based coupling reagent is 4-(4,6-dimethoxy-1,3,5-triazin-2-yl)-4-methylmorpholinium chloride (DMTMM); or wherein the triazine-based coupling reagent is 2-chloro-4,6-dimethoxy-1,3,5-triazin (CDMT) together with N-methylmorpholine (NMM).

5. A process according to any one of claims 1-4, wherein the chondroitin molecules are chondroitin sulfate.

6. A hydrogel product comprising a cross-linked network of chondroitin molecules selected from the group consisting of chondroitin and chondroitin sulfate as the swellable polymer, wherein the carboxyl groups on the chondroitin molecules are activated using a triazine-based coupling reagent and wherein the chondroitin molecules are cross-linked to each other via their carboxyl groups using a cross-linking agent, essentially without any N-acylurea groups on the chondroitin molecules; wherein the crosslinking agent is hexamethylenediamine (HMDA).

7. A hydrogel product according to claim 6, wherein the chondroitin molecule is cross-linked by amide bonds.

8. A hydrogel product according to any one of claims 6-7, wherein the chondroitin molecules are chondroitin sulfate.

9. A hydrogel product according to any one of claims 6-8 for use as a medicament, such as in the treatment of soft tissue disorders.

## Patentansprüche

1. Verfahren zur Herstellung eines Hydrogelprodukts, das ein vernetztes Netzwerk von Chondroitinmolekülen umfasst, bei dem man:
(a) Chondroitinmoleküle aus der Gruppe bestehend aus Chondroitin und Chondroitinsulfat bereitstellt;
(b) die Carboxylgruppen der Chondroitinmoleküle mit einem auf Triazin basierenden Kupplungsreagenz aktiviert, was ein aktiviertes, vernetztes Chondroitin im Wesentlichen ohne jegliche N-Acylharnstoffgruppen ergibt;
(c) die aktivierten Chondroitinmoleküle unter Verwendung eines Vernetzungsmittels über ihre Carboxylgruppen miteinander vernetzt, wobei es sich bei dem Vernetzungsmittel um Hexamethylendiamin (HMDA) handelt.

2. Verfahren nach Anspruch 1, wobei die Vernetzung von Schritt (c) Amidbindungen liefert.

3. Verfahren nach einem der Ansprüche 1-2, wobei das auf Triazin basierende Kupplungsreagenz aus 4,6-disubstituierten 2-Chlor-1,3,5-triazinen in Gegenwart einer tertiären Aminbase oder der entsprechenden quaternären Ammoniumsalze davon ausgewählt wird.

4. Verfahren nach Anspruch 3, wobei es sich bei dem auf Triazin basierenden Kupplungsreagenz um 4-(4,6-Dimethoxy-1,3,5-triazin-2-yl)-4-methylmorphol-iniumchlorid (DMTMM) handelt oder wobei es sich bei dem auf Triazin basierenden Kupplungsreagenz um 2-Chlor-4,6-dimethoxy-1,3,5-triazin (CDMT) zusammen mit N-Methylmorpholin (NMM) handelt.

5. Verfahren nach einem der Ansprüche 1-4, wobei es sich bei den Chondroitinmolekülen um Chondroitinsulfat handelt.

6. Hydrogelprodukt, umfassend ein vernetztes Netzwerk von Chondroitinmolekülen aus der Gruppe bestehend aus Chondroitin und Chondroitinsulfat als quellbares Polymer, wobei die Carboxylgruppen an den Chondroitinmolekülen unter Verwendung eines auf Triazin basierenden Kupplungsreagenz aktiviert sind und wobei die Chondroitinmoleküle unter Verwendung eines Vernetzungsmittels über ihre Carboxylgruppen im Wesentlichen ohne jegliche N-Acylharnstoffgruppen an den Chondroitinmolekülen miteinander vernetzt sind; wobei es sich bei dem Vernetzungsmittel um Hexamethylendiamin (HMDA) handelt.

7. Hydrogelprodukt nach Anspruch 6, wobei das Chondroitinmolekül durch Amidbindungen vernetzt ist.

8. Hydrogelprodukt nach einem der Ansprüche 6-7, wobei es sich bei den Chondroitinmolekülen um Chondroitinsulfat handelt.

9. Hydrogelprodukt nach einem der Ansprüche 6-8 zur Verwendung als Medikament, wie bei der Behandlung von Weichteilstörungen.

## Revendications

1. Procédé de préparation d'un produit de type hydrogel comprenant un réseau réticulé de molécules de chondroïtine, comprenant les étapes de :
(a) mise à disposition de molécules de chondroïtine choisies dans le groupe constitué par la chondroïtine et le sulfate de chondroïtine ;
(b) activation des groupes carboxyle sur les molécules de chondroïtine avec un réactif de couplage à base de triazine pour former une chondroïtine réticulée, activée, essentiellement sans aucun groupe de type N-acylurée ;
(c) réticulation des molécules de chondroïtine activées l'une avec l'autre via leurs groupes carboxyle à l'aide d'un agent de réticulation, l'agent de réticulation étant l'hexaméthylènediamine (HMDA).

2. Procédé selon la revendication 1, la réticulation de l'étape (c) fournissant des liaisons amide.

3. Procédé selon l'une quelconque des revendications 1 et 2, le réactif de couplage à base de triazine étant choisi parmi des 2-chloro-1,3,5-triazines 4,6-disubstituées en présence d'une base de type amine tertiaire et des sels d'ammonium quaternaires correspondants de celles-ci.

4. Procédé selon la revendication 3, le réactif de couplage à base de triazine étant le chlorure de 4-(4,6-diméthoxy-1,3,5-triazin-2-yl)-4-méthylmorpholinium (DMTMM) ; ou le réactif de couplage à base de triazine étant la 2-chloro-4,6-diméthoxy-1,3,5-triazine (CDMT) conjointement avec la N-méthylmorpholine (NMM).

5. Procédé selon l'une quelconque des revendications 1 à 4, les molécules de chondroïtine étant le sulfate de chondroïtine.

6. Produit de type hydrogel comprenant un réseau réticulé de molécules de chondroïtine choisies dans le groupe constitué par la chondroïtine et le sulfate de chondroïtine en tant que polymère gonflable, les groupes carboxyle sur les molécules de chondroïtine étant activés à l'aide d'un réactif de couplage à base de triazine et les molécules de chondroïtine étant réticulées l'une avec l'autre via leurs groupes carboxyle à l'aide d'un agent de réticulation, essentiellement sans aucun groupe de type N-acylurée sur les molécules de chondroïtine ; l'agent de réticulation étant l'hexaméthylènediamine (HMDA).

7. Produit de type hydrogel selon la revendication 6, les molécules de chondroïtine étant réticulées par des liaisons amide.

8. Produit de type hydrogel selon l'une quelconque des revendications 6 et 7, les molécules de chondroïtine étant le sulfate de chondroïtine.

9. Produit de type hydrogel selon l'une quelconque des revendications 6 à 8 pour une utilisation en tant que médicament, telle que dans le traitement de troubles des tissus mous.
